# EUROPEAN PATENT APPLICATION

(11) **EP 4 368 181 A1**
(43) Date of publication of application: **15.05.2024**
(21) Application number: 22837752.9
(22) Date of filing: 07.07.2022
(51) Int. Cl.: A61K 31/39, A61K 35/30, A61L 27/38, A61L 27/50, C12N 5/071

(54) **CELL SEEDING AGENT AND SUBSTRATE FOR CELL TRANSPLANTATION USE**

(30) Priority: 07.07.2021 US 202163219101 P
(71) Applicant: Cellusion Inc., Tokyo 103-0024 (JP)
(72) Inventor: YOSHIZAKI, Shinji, Tokyo 103-0022 (JP); HATOU, Shin, Tokyo 103-0022 (JP); SAKAGUCHI, Yoshiki, Tokyo 103-0022 (JP)
(74) Representative: J A Kemp LLP
(86) International application number: PCT/JP2022/027003
(87) International publication number: WO 2023/282338

(57) **Abstract**

The present invention aims to provide a cell seeding agent and a substrate for transplantation that can perform passage operation without causing cell transformation and can efficiently engraft cells. A seeding agent for promoting the engraftment of a cultured corneal endothelial cell or a corneal endothelial substitute cell, containing a phenolphthalein derivative, and a substrate for transplantation. Such seeding agent and substrate for transplantation can promote cell adhesion during passage operations and cell engraftment during transplantation.

## Description

### [Technical Field]

The present invention relates to a cell seeding agent and a substrate for cell transplantation, particularly, a seeding agent for and a substrate for transplantation of a corneal endothelial cell or a corneal endothelial substitute cell.

### [Background Art]

Corneal endothelial damage, such as decrease in corneal endothelial cells, impairs functions of corneal endothelial cell and causes edema in the corneal stroma. This decreases transparency of the cornea, and reduces the visual acuity. Such condition is called bullous keratopathy. In the meantime, it is known that human corneal endothelial cell once injured scarcely shows an ability to regenerate. When the corneal endothelial cells have decreased due to certain injury, an effective or sole treatment thereof is corneal transplantation. In fact, about half the number of applicable cases of corneal transplantation is for bullous keratopathy caused by corneal endothelial functional disorder.

At present, patients with corneal endothelium damage are treated by penetrating keratoplasty wherein the whole three-layer structure of corneal epithelium, corneal stroma and corneal endothelium is transplanted. While the penetrating keratoplasty is an established technique, the supply of cornea is short in Japan as the situation stands, and the rejection reaction poses a problem. To solve such problems, "part transplantation" involving transplantation of only the damaged tissue is becoming popular. Deep lamellar keratoplasty (DLKP) involving transplantation of only the epithelium and stroma of the donor while preserving corneal endothelium, corneal endothelium transplantation involving transplantation of only the part cornea including endothelium and the like are known. However, in the case of corneal endothelium transplantation, for example, the source of supply of the material for transplantation is still the corneal endothelium itself. Since the number of donor of cornea is limited, the problem of donor shortage cannot be overcome, like penetrating keratoplasty. Furthermore, since corneal endothelial cell is difficult to culture, preparation of cultured cells in a number sufficient for transplantation places a large burden in terms of time and cost.

In order to solve the problem of donor shortage, attempts are being made to create corneal endothelial cells or cells that have functions equivalent to those of corneal endothelial cells and can replace corneal endothelial cells.

For example, methods for inducing cells that can replace corneal endothelial cells from iPS cells or ES cells have been reported. Comprehensive review of the methods for inducing corneal endothelial cells (actually, since surface markers specific to corneal endothelial cells have not been established so far, cells with properties similar to those of corneal endothelial cells, so-called corneal endothelial like cells) from iPS cells or ES cells and cell culture methods is provided in Non Patent Literature 1. In addition, the present inventors have so far established a method for producing cells that have functions equivalent to those of corneal endothelial cells and can replace corneal endothelial cells (Patent Literatures 1 to 3), and named the corneal endothelial-like cell obtained by this method as "corneal endothelial substitute cell ". Clinical research on iPS cell -derived corneal endothelial substitute cell for bullous keratopathy has also begun.

### [Citation List]

### [Patent Literature]

[Patent Literature 1]
   WO/2013/051722
[Patent Literature 2]
   WO/2016/093359
[Patent Literature 3]
   WO/2019/142833

### [Non Patent Literature]

[Non Patent Literature 1]
Hatou S, Shimmura S; Inflamm Regen. 2019; 39:19.

### [Summary of Invention]

### [Technical Problem]

In order to perform large-scale production of target cells, particularly corneal endothelial substitute cells derived from iPS cells or ES cells, and obtain final products from them, it is necessary not only for cells to proliferate, but also to engraft cells as efficiently as possible during the passage operation (collecting cells from a cell culture container and reseeding them into a new cell culture container) across each step. In addition, in order to perform the passage operation without causing cell transformation, the components contained in the medium during the passage operation are important. In addition, the operation of administering a cell suspension into the anterior chamber of the eye and achieving uniform engraftment on the posterior cornea (endothelial surface) is an in vivo passage operation. Like the medium used during cell seeding, the components contained in the transplantation substrate are important.

In the passage operation in the expansion culture step of iPS cells, a method including temporarily adding a ROCK inhibitor represented by Y-27632 to the medium is used. However, the present inventors have found that use of a ROCK inhibitor in the step of inducing differentiation from iPS cells into corneal endothelial substitute cells inhibits formation of cytoskeleton and tight junctions, resulting in transformation that appears to be epithelial-to-mesenchymal transition (EMT). When EMT occurs, the functions of corneal epithelial cells as epithelial cells (e.g., barrier function) decrease.

Therefore, the present invention aims to provide a cell seeding agent and a substrate for cell transplantation that can perform passage operation without causing cell transformation and can efficiently engraft cells.

### [Solution to Problem]

The present inventors have conducted intensive studies in view of the above-mentioned problems and found that the engraftment of cells can be efficiently promoted even in the absence of a ROCK inhibitor, by including a phenolphthalein derivative during passage operations when inducing corneal endothelial cells or corneal endothelial substitute cells from iPS cells or ES cells, and that also in a substrate for cell transplantation, the phenolphthalein derivative promotes uniform engraftment on the posterior cornea, which consequently improves the treatment effect of the transplanted cells, and completed the present invention.

That is, the present invention provides the following.
[1] A seeding agent for promoting the engraftment of a cultured corneal endothelial cell or a corneal endothelial substitute cell, comprising a phenolphthalein derivative.
[2] A seeding agent for promoting the engraftment of a corneal endothelial substitute cell, comprising a phenolphthalein derivative.
[3] The agent of the above-mentioned [2], wherein the corneal endothelial substitute cell is derived from a stem cell.
[4] The agent of the above-mentioned [3], wherein the stem cell is a pluripotent stem cell.
[5] The agent of the above-mentioned [4], wherein the pluripotent stem cell is derived from an iPS cell (including a cell imparted with an additional function by gene editing or gene transfer).
[6] The agent of any of the above-mentioned [1] to [5], wherein the phenolphthalein derivative is selected from the group consisting of phenolsulfonphthalein (phenol red), isovalerylphenolphthalein, acetylphenolphthalein, phenolphthalein dibutyrate, phenolphthalein diphosphate, phenolphthalein disulfate, phenolphthalein glucuronic acid, phenolphthalein mono-β-glucosiduronic acid, phenolphthalein mono-β-glucuronic acid, phenolphthalein mono-phosphate, cresol red, thymol blue, and bromocresol purple.
[7] The agent of any of the above-mentioned [1] to [6], wherein the phenolphthalein derivative is phenol red.
[8] The agent of the above-mentioned [7], wherein a concentration of the phenol red is 1 to 20 mg/L.
[9] The agent of any of the above-mentioned [1] to [6], wherein the phenolphthalein derivative is cresol red.
[10] The agent of the above-mentioned [9], wherein a concentration of the cresol red is 1 to 20 mg/L.
[11] The agent of any of the above-mentioned [1] to [10], which is free of a ROCK inhibitor.
[12] Use of a phenolphthalein derivative for the production of a seeding agent for promoting engraftment of a cultured corneal endothelial cell or a corneal endothelial substitute cell.
[13] Use of a phenolphthalein derivative for the production of a seeding agent for promoting engraftment of a corneal endothelial substitute cell.
[14] The use of the above-mentioned [12] or [13], wherein the corneal endothelial substitute cell is derived from a stem cell.
[15] The use of the above-mentioned [14], wherein the stem cell is a pluripotent stem cell.
[16] The use of the above-mentioned [15], wherein the pluripotent stem cell is derived from an iPS cell (including a cell imparted with an additional function by gene editing or gene transfer).
[17] The use of any of the above-mentioned [12] to [16], wherein the phenolphthalein derivative is selected from the group consisting of phenolsulfonphthalein (phenol red), isovalerylphenolphthalein, acetylphenolphthalein, phenolphthalein dibutyrate, phenolphthalein diphosphate, phenolphthalein disulfate, phenolphthalein glucuronic acid, phenolphthalein mono-β-glucosiduronic acid, phenolphthalein mono-β-glucuronic acid, phenolphthalein mono-phosphate, cresol red, thymol blue, and bromocresol purple.
[18] The use of any of the above-mentioned [12] to [17], wherein the phenolphthalein derivative is phenol red.
[19] The use of any of the above-mentioned [12] to [17], wherein the phenolphthalein derivative is cresol red.
[20] A phenolphthalein derivative for use in promoting the engraftment of a cultured corneal endothelial cell or a corneal endothelial substitute cell when seeding the cell.
[21] A phenolphthalein derivative for use in promoting the engraftment of a corneal endothelial substitute cell when seeding the cell.
[22] The derivative of the above-mentioned [20] or [21], wherein the corneal endothelial substitute cell is derived from a stem cell.
[23] The derivative of the above-mentioned [22], wherein the stem cell is a pluripotent stem cell.
[24] The derivative of the above-mentioned [23], wherein the pluripotent stem cell is derived from an iPS cell (including a cell imparted with an additional function by gene editing or gene transfer).
[25] The derivative of any of the above-mentioned [20] to [24], wherein the phenolphthalein derivative is selected from the group consisting of phenolsulfonphthalein (phenol red), isovalerylphenolphthalein, acetylphenolphthalein, phenolphthalein dibutyrate, phenolphthalein diphosphate, phenolphthalein disulfate, phenolphthalein glucuronic acid, phenolphthalein mono-β-glucosiduronic acid, phenolphthalein mono-β-glucuronic acid, phenolphthalein mono-phosphate, cresol red, thymol blue, and bromocresol purple.
[26] The derivative of any of the above-mentioned [20] to [25], wherein the phenolphthalein derivative is phenol red.
[27] The derivative of any of the above-mentioned [20] to [25], wherein the phenolphthalein derivative is cresol red.
[28] A method for promoting the engraftment of a cultured corneal endothelial cell or a corneal endothelial substitute cell, comprising seeding the cell in the presence of a phenolphthalein derivative.
[29] A method for promoting the engraftment of a corneal endothelial substitute cell, comprising seeding the cell in the presence of a phenolphthalein derivative.
[30] The method of the above-mentioned [28] or [29], wherein the corneal endothelial substitute cell is derived from a stem cell.
[31] The method of the above-mentioned [30], wherein the stem cell is a pluripotent stem cell.
[32] The method of the above-mentioned [31], wherein the pluripotent stem cell is derived from an iPS cell (including a cell imparted with an additional function by gene editing or gene transfer).
[33] The method of any of the above-mentioned [28] to [32], wherein the phenolphthalein derivative is selected from the group consisting of phenolsulfonphthalein (phenol red), isovalerylphenolphthalein, acetylphenolphthalein, phenolphthalein dibutyrate, phenolphthalein diphosphate, phenolphthalein disulfate, phenolphthalein glucuronic acid, phenolphthalein mono-β-glucosiduronic acid, phenolphthalein mono-β-glucuronic acid, phenolphthalein mono-phosphate, cresol red, thymol blue, and bromocresol purple.
[34] The method of any of the above-mentioned [28] to [33], wherein the phenolphthalein derivative is phenol red.
[35] The method of any of the above-mentioned [28] to [33], wherein the phenolphthalein derivative is cresol red.
[36] A cell suspension comprising the agent of any of the above-mentioned [2] to [11] and a corneal endothelial substitute cell.
[37] The cell suspension of the above-mentioned [36], wherein a concentration of the cell is 1.0 to 20×10⁴ cells/mL.
[38] The cell suspension of the above-mentioned [36] or [37], wherein the seeding is performed at a seeding density of not less than 1.04×10³ cells/cm² and less than 1.04×10⁴ cells/cm².
[39] A substrate for the transplantation of a cultured corneal endothelial cell or a corneal endothelial substitute cell, comprising a phenolphthalein derivative.
[40] A substrate for the transplantation of a corneal endothelial substitute cell, comprising a phenolphthalein derivative.
[41] The substrate of the above-mentioned [39] or [40], wherein the corneal endothelial substitute cell is derived from a stem cell.
[42] The substrate of the above-mentioned [41], wherein the stem cell is a pluripotent stem cell.
[43] The substrate of the above-mentioned [42], wherein the pluripotent stem cell is derived from an iPS cell (including a cell imparted with an additional function by gene editing or gene transfer).
[44] The substrate of any of the above-mentioned [39] to [43], wherein the phenolphthalein derivative is selected from the group consisting of phenolsulfonphthalein (phenol red), phenolphthalein, bromophenol blue, isovalerylphenolphthalein, acetylphenolphthalein, phenolphthalein dibutyrate, phenolphthalein diphosphate, phenolphthalein disulfate, phenolphthalein glucuronic acid, phenolphthalein mono-β-glucosiduronic acid, phenolphthalein mono-β-glucuronic acid, phenolphthalein mono-phosphate, cresol red, thymol blue, and bromocresol purple.
[45] The substrate of any of the above-mentioned [39] to [44], wherein the phenolphthalein derivative is phenol red.
[46] The substrate of the above-mentioned [45], wherein a concentration of the phenol red is 1 to 20 mg/L.
[47] The substrate of any of the above-mentioned [39] to [44], wherein the phenolphthalein derivative is cresol red.
[48] The substrate of the above-mentioned [47], wherein a concentration of the cresol red is 1 to 20 mg/L.
[49] The substrate of any of the above-mentioned [39] to [48], which is free of a ROCK inhibitor.
[50] A cell suspension comprising the substrate of any of the above-mentioned [39] to [49] and a corneal endothelial substitute cell.
[51] The cell suspension of the above-mentioned [50], which is for transplantation into the anterior chamber.
[52] The cell suspension of the above-mentioned [51], wherein a concentration of the cell is 0.5 to 10×10⁶ cells/mL.

### [Advantageous Effects of Invention]

According to the present invention, it is possible to perform passage operation without causing cell transformation and efficiently engraft cells. Large-scale production of cultured corneal endothelial cells and corneal endothelial substitute cells can be achieved, and the engraftment rate of transplanted cells is increased, thereby making it possible to improve treatment effects.

### [Brief Description of Drawings]

[Fig. 1]

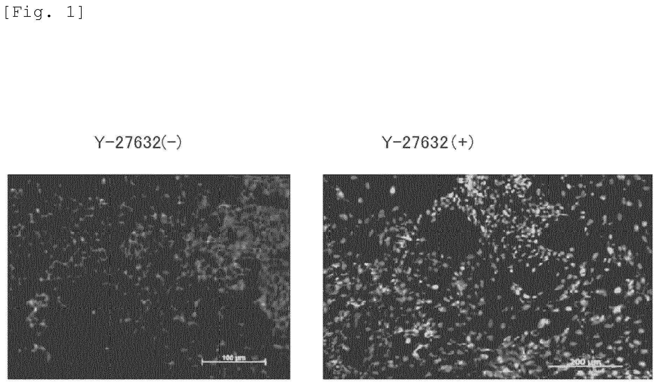

   Fig. 1 is a diagram showing the results of observing formation of tight junction with and without the use of ROCK inhibitor (Y-27632) during the passage operation in the differentiation induction step of iPS cell-derived corneal endothelial substitute cells. When the ROCK inhibitor was used, formation of tight junction was inhibited.
[Fig. 2]

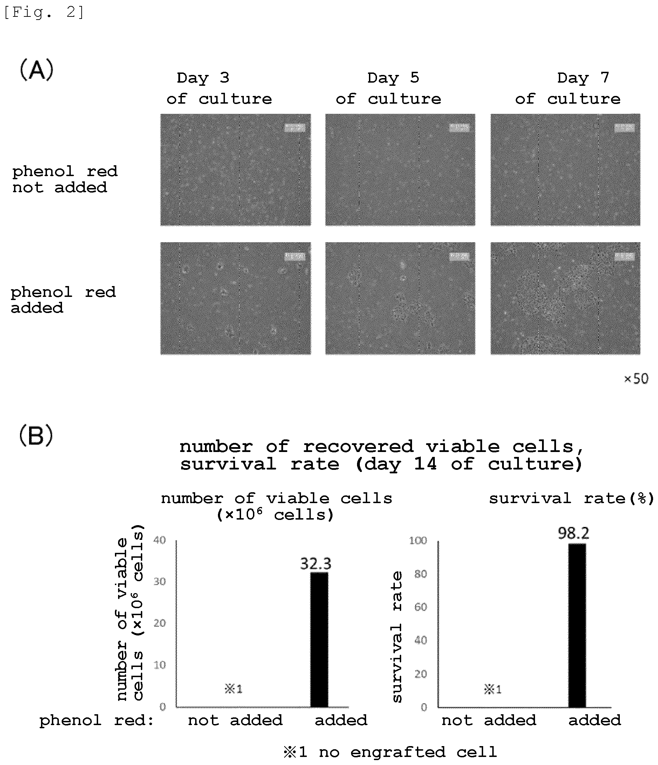

   Fig. 2 is a diagram showing the investigation results of the influence of phenolphthalein derivatives (phenol red) during the passage operation in the differentiation induction step of iPS cell-derived corneal endothelial substitute cells. The upper panel (A) is a microscopic photograph of the state of cells observed on the 3rd, 5th, and 7th day after culturing in the presence or absence of phenol red. The lower panel (B) is a graph showing the results of examining the number and survival rate of the cells collected on day 14 of culture in the presence or absence of phenol red.
[Fig. 3]

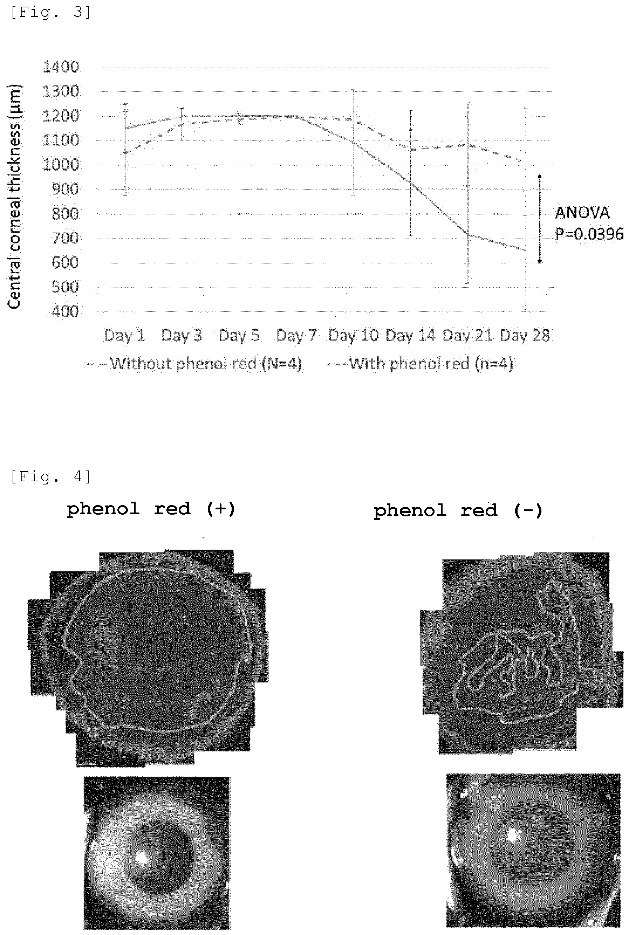

   Fig. 3 is a graph showing the results of measuring corneal thickness when iPS cell-derived corneal endothelial substitute cells were transplanted in the presence or absence of phenol red using a Macaca fascicularis bullous keratopathy model. The corneal thickness decreased and corneal edema-improving effect was observed by transplantation in the presence of phenol red.
[Fig. 4]
   Fig. 4 shows the results of observing the distribution of corneal endothelial substitute cells in the anterior chamber of the eye when iPS cell-derived corneal endothelial substitute cells were transplanted in the presence or absence of phenol red using a Macaca fascicularis bullous keratopathy model.

### [Description of Embodiments]

The present invention is explained in the following. Unless particularly indicated, the terms used in the present specification have the meanings generally used in the pertinent field.

### 1. Cell seeding agent and substrate for cell transplantation

The present invention provides a novel cell seeding agent. The cell seeding agent refers to, in a cell culture technique, a liquid (solution, suspension, etc.) used to mix cells when seeding cells into a new culture container during a passage operation.

More particularly, the present invention provides a cell seeding agent containing a phenolphthalein derivative, which is used in a step of inducing corneal endothelial substitute cells from iPS cells or ES cells. The cell seeding agent is preferably a seeding agent for promoting cell engraftment. Preferably, it is a seeding agent containing a phenolphthalein derivative for promoting engraftment of corneal endothelial substitute cells. In the present specification, these are sometimes collectively abbreviated as the cell seeding agent of the present invention.

In addition, the present invention provides a novel substrate for transplantation. A substrate for transplantation means refers to a liquid (solution, suspension, etc.) used to administer cells into a human body or an animal body.

More particularly, the present invention provides a substrate for the transplantation of a cultured corneal endothelial cell or a corneal endothelial substitute cell, comprising a phenolphthalein derivative. Preferably, it is a substrate containing a phenolphthalein derivative for transplantation of corneal endothelial substitute cells. In the present specification, these are sometimes collectively abbreviated as the substrate for cell transplantation of the present invention.

The cell seeding agent of the present invention is preferably used in a passage operation (collecting cells from a cell culture container and reseeding them into a new cell culture container) of cultured corneal endothelial cells or corneal endothelial substitute cells, preferably corneal endothelial substitute cells. The substrate for cell transplantation of the present invention is preferably used when transplanting cultured corneal endothelial cells or corneal endothelial substitute cells, preferably corneal endothelial substitute cells into the anterior chamber of the eye.

### (Cell)

The cell to be the target for the cell seeding agent or substrate for cell transplantation of the present invention is a cultured corneal endothelial cell or a corneal endothelial substitute cell, preferably a corneal endothelial substitute cell, more preferably a stem cell-derived corneal endothelial substitute cell. As used herein, the stem cell is preferably a pluripotent stem cell, more preferably an iPS cell.

While the cultured corneal endothelial cells may be primary cultured cells or established line of cells, established line of cells are preferably used since primary culture of corneal endothelial cells is difficult.

In the present invention, the "corneal endothelial substitute cell" is a cell induced from a stem cell such as iPS cell or ES cell, or such cell imparted with additional functions through gene editing or gene transfer, and the like, and can treat a corneal endothelium functional disorder and replace corneal endothelial cells. That is, the corneal endothelial substitute cell has physiological functions equivalent to those of the corneal endothelial cell.

The "stem cell" means a cell capable of being cultured in vitro, and capable of differentiating into plural lines of cells constituting the body. Specific examples include embryonic stem cell (ES cell), fetal primordial germ cell-derived pluripotent stem cell (EG cell), testis-derived pluripotent stem cell (GS cell), somatic cell-derived artificial pluripotent stem cell (induced pluripotent stem cells; iPS cell), and human somatic stem cell (tissue stem cell). As the iPS cell, an iPS cell derived from any warm-blooded animal, preferably a mammal, can be used. Examples of the mammal include mouse, rat, guinea pig, hamster, rabbit, cat, dog, sheep, swine, bovine, horse, goat, monkey, human and the like. A cell derived from human can be preferably used.

Specific examples of the iPS cell include cells obtained by introducing plural genes into a somatic cell such as skin cell and the like, which have acquired multipotency same as that of ES cell. Examples thereof include iPS cell obtained by introducing Oct3/4 gene, Klf4 gene, C-Myc gene and Sox2 gene, iPS cell obtained by introducing Oct3/4 gene, Klf4 gene and Sox2 gene (Nat Biotechnol 2008; 26: 101-106) and the like. Furthermore, the production method of iPS cell has been intensively improved technically, for example, a method involving further reducing transgenes (Nature. 2008 Jul 31; 454(7204): 646-50), a method utilizing a low-molecular-weight compound (Cell Stem Cell. 2009 Jan 9; 4(1): 16-9, Cell Stem Cell. 2009 Nov 6; 5(5): 491-503), a method utilizing a transcription factor protein instead of the gene (Cell Stem Cell. 2009 May 8; 4(5): 381-4) and the like. Since the basic property of the iPS cells produced, that is, having multipotency, is equivalent irrespective of the production methods, all of them can be the origin of the corneal endothelial substitute cell used in the present invention.

Specifically, as the iPS cell, 201B7, 201B7-Ff, 253G1, 253G4, 1201C1, 1205D1, 1210B2, 836B3, FF-I14s03, FF-I01s04, MH09s01, Ff-XT18s02, Ff-WIs03, Ff-WJs513, Ff-CLs14, Ff-KVs09, QHJI14s03, QHJI01s04, RWMH09s01, DRXT18s02, RJWIs03, YZWJs513, ILCLs14, GLKVs09, Ff-XT28s05-ABo_To, Ff-I01s04-ABII-KO, Ff-I14s04-ABII-KO (all iPS Academia Japan, Inc., or CiRA Foundation), Tic (JCRB1331 strain), Dotcom (JCRB1327 strain), Squeaky (JCRB1329 strain), and Toe (JCRB1338 strain), Lollipop (JCRB1336 strain) (National Center for Child Health and Development, National Institutes of Biomedical Innovation, Department of Intractable Diseases and Disease Resources, JCRB cell Bank), UTA-1 strain and UTA-1-SF-2-2 strain (all The University of Tokyo), 21526, 21528, 21530, 21531, 31536, 31538 strains (all FUJIFILM Cellular Dynamics, Inc.), and the like can be used.

A corneal endothelial substitute cell to be the target in the cell seeding agent or the substrate for cell transplantation of the present invention is, for example, a corneal endothelial-like cell developed by the present inventors (Patent Literatures 1 to 3), and can be produced and prepared by the methods described in Patent Literatures 1 to 3. Preferably, it is a corneal endothelial substitute cell derived from an iPS cell (including cells imparted with additional functions through gene editing or transfection) (Corneal Endothelial Cell Substitute from iPS Cells; CECSi cell), which has properties and functions similar to those of corneal endothelial cells and has enhanced gene expression level of NR3C2 (nuclear receptor subfamily 3, group C, member 2) (Patent Literature 3).

The corneal endothelial cell-like properties and functions that the corneal endothelial substitute cell has specifically include the following characteristics (i) to (iv). The cell has at least one, preferably two, more preferably three, and even more preferably all four, of these characteristics.
(i) Intercellular adhesion is composed of N-Cadherin.
(ii) Tight junction is formed between cells.
(iii) Na,K-ATPase α1 subunit is expressed on the cell membrane.
(iv) Expression of transcription factor PITX2 is observed in the cell nucleus.

Whether or not intercellular adhesion is composed of N-Cadherin can be confirmed by immunostaining for N-Cadherin.

Whether or not tight junction is formed between cells can be confirmed by observing the presence of ZO-1, which is a protein constituting tight junctions, by immunostaining for ZO-1. It can also be confirmed by directly observing the structure using an electron microscope.

Whether or not Na,K-ATPase α1 subunit (ATP1A1) is expressed on the cell membrane can be confirmed by co-staining of ZO-1 and Na,K-ATPase α1 subunit by immunostaining.

Whether or not the transcription factor PITX2 is expressed in the cell nucleus can be confirmed by immunostaining for PITX2.

In one embodiment of the present invention, a corneal endothelial substitute cell to be the target in the cell seeding agent or the substrate for cell transplantation of the present invention is characterized by having a CD24-positive phenotype. CD24 is a highly glycosylated, small mucin-like glycosylphosphatidyl-inositol (GPI)-binding cell surface protein. CD24 is highly expressed on blood-lineage cells such as B cells, T cells, neutrophils, eosinophils, dendritic cells, and macrophages, as well as non-blood-lineage cells such as neurons, ganglion cells, epithelial cells, keratinocytes, muscle cells, pancreatic cells, and epithelial stem cells. In recent years, it has been reported that CD24 has the function of avoiding phagocytosis by macrophages. Therefore, the cell seeding agent or substrate for cell transplantation of the present invention containing corneal endothelial substitute cell having a CD24-positive phenotype can be expected to reduce rejection reactions in transplantation. On the other hand, it has been reported that human corneal endothelial cells are CD24 negative (JP-A-2019-510509, JP-A-2020-073602).

The presence or absence of a CD24-positive phenotype can be confirmed by immunostaining for the CD24 antigen expressed on the cell surface.

Each immunostaining is generally performed in the art, and the reagents, equipment, and the like to be used are commercially available or can be prepared according to previous reports.

In the passage operation in the step of inducing differentiation of the above-mentioned corneal endothelial substitute cells, stem cells such as iPS cells, ES cells, or cells imparted with additional functions by gene editing or gene transfer, and the like are added to a cell seeding agent, and a cell suspension obtained by suspending to a concentration of generally 1.0 to 20×10⁴ cells/mL, preferably 2.5 to 10×10⁴ cells/mL, is seeded in a culture vessel such as a culture dish at a seeding density of not less than 1.04×10³ cells/cm² and less than 1.04×10⁴ cells/cm², preferably not less than 1.04×10³ cells/cm² and not more than 5.02×10³ cells/cm².

In the process of transplanting into the eye, particularly into the anterior chamber of the eye, the above-mentioned cultured corneal endothelial cells or corneal endothelial substitute cells, preferably corneal endothelial substitute cells, are added to a substrate for cell transplantation, and a cell suspension obtained by suspending to a concentration of generally 0.5 to 10×10⁶ cells/mL, preferably 1.0 to 5×10⁶ cells/mL, is transplanted. Cell engraftment efficiency decreases if the cell concentration and/or seeding density are/is too high or too low.

In the present invention, the cultured corneal endothelial cell or corneal endothelial substitute cell may be a spherical cell mass obtained by aggregation of several dozens to several hundreds of cells to form a spheroid by suspension culture (hereinafter to be also simply referred to as spheroid). As used herein, "spherical" includes a complete sphere, as well as substantially spherical shapes such as an egg shape and a rugby ball shape. The spheroid has a diameter or area equivalent diameter in the range of preferably 10 to 200 µm, more preferably in the range of 30 to 150 um, particularly preferably in the range of 30 to 100 µm, even more preferably in the range of 30 to 90 um, and even further preferably in the range of 40 to 80 um. When the spheroid is too large, engraftment becomes uneven and not uniform, and when the spheroid is too small, engraftment becomes difficult and the engraftment efficiency decreases. When the size of the spheroid is within this range, cells are efficiently engrafted at the transplantation site. The "area equivalent diameter" refers to the diameter of a circle that is equal to the projected area of a particle (in the present case, a spheroid), and is also referred to as the Heywood diameter or equivalent circle diameter. That is, it is the diameter of a circle having an area equal to the projected area of a substantially globular spheroid.

Cell concentration and seeding density is determined by sampling a part of the cell suspension and counting the cells in the sample; and when the dosage form is a spheroid suspension, cell concentration is determined by sampling a part of the spheroid suspension, dispersing the cells into single cells by an enzyme treatment of the spheroid in the sample, counting the cells in the sample and using the results thereof.

### (Phenolphthalein derivative)

The cell seeding agent or substrate for cell transplantation of the present invention is characterized in that it contains a phenolphthalein derivative. The phenolphthalein derivative is not particularly limited as long as it has the basic skeleton represented by the following formula to achieve the desired effect. 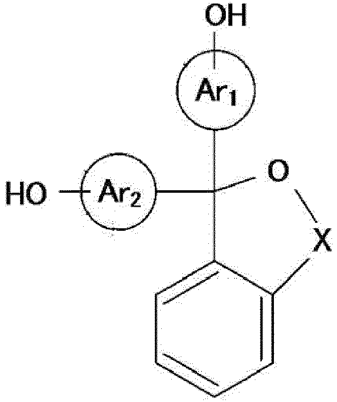 wherein -X- is -SO₂- or -CO-, and ring Ar₁ and ring Ar₂ are the same or different and each is an aromatic ring having 6 or 12 carbon atoms (e.g., benzene, naphthalene) optionally having 1 to 3 substituents (e.g., alkyl groups optionally having a branched chain having 1 to 3 carbon atoms such as methyl, ethyl, propyl, isopropyl and the like; halogen atoms such as bromine atom, chlorine atom, and the like; acyl groups such as acetyl, isovaleryl, and the like).

Preferably, -X- is -SO₂-. Ring Ar₁ and ring Ar₂ are the same or different and each is benzene optionally having 1 to 3, preferably 1 or 2 substituents (e.g., methyl, isopropyl, bromine atom).

The phenolphthalein derivative may be a salt thereof or an ester thereof. Alternatively, it may be an acid adduct.

Examples of the phenolphthalein derivative include phenolsulfonphthalein (phenol red), phenolphthalein, bromophenol blue, isovalerylphenolphthalein, acetylphenolphthalein, phenolphthalein dibutyrate, phenolphthalein diphosphate, phenolphthalein disulphate, phenolphthalein glucuronic acid, phenolphthalein mono-β-glucosiduronic acid, phenolphthalein mono-β-glucuronic acid, phenolphthalein mono-phosphate, cresol red, thymol blue, and bromocresol purple. The phenolphthalein derivative can be produced by a method known per se. It is known that phenolphthalein derivatives can be synthesized from phthalic acid and various corresponding phenols, and when the phenols used is phenol, phenolphthalein can be synthesized. Also, various phenolphthalein derivatives are commercially available. From the viewpoint of convenience and stability of quality, it is preferable to use commercially available products. Preferably, the phenolphthalein derivative is phenol red (also to be referred to as phenolsulfonphthalein) (left) or cresol red (right) having the following structures. Phenol red is generally included in a medium as a pH indicator with a color change range (yellow to red) of pH 6.8 to pH 8.4. It is an optional addition component from the viewpoint of cell culture, and a phenol red-free medium is also commercially available. Cresol red is also known as a pH indicator with a color change (yellow to red) range of pH 7.2 to pH 8.8. The concentration of phenol red contained in cell seeding agent or substrate for cell transplantation of the present invention is generally 1 to 20 mg/L, preferably 1 to 15 mg/L, more preferably 5 to 10 mg/L. The concentration of cresol red contained in the cell seeding agent or substrate for cell transplantation of the present invention during differentiation induction is generally 1 to 20 mg/L, preferably 1 to 15 mg/L, more preferably 5 to 10 mg/L. At the time of transplantation, it is 1 to 16 mg/L, preferably 1 to 12 mg/L, more preferably 4 to 8 mg/L. The concentration of phenolphthalein derivative other than phenol red cresol red is also generally 1 to 20 mg/L, preferably 1 to 15 mg/L, more preferably 5 to 10 mg/L.

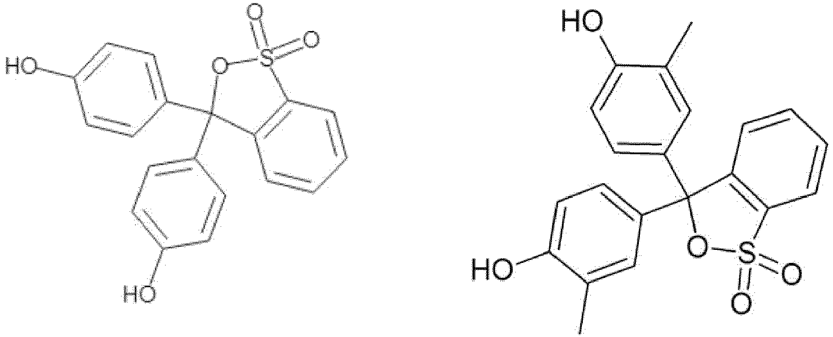

The "desired effect" of the phenolphthalein derivative is the effect of promoting engraftment of cells after passage operation or after transplantation.

Examples of the subject of administration of the substrate for cell transplantation of the present invention include mammals such as mouse, rat, hamster, guinea pig, rabbit, cat, dog, bovine, horse, sheep, monkey, human and the like, and preferred is human.

The dose of the substrate for cell transplantation of the present invention varies depending on the body weight, age, symptom and the like of the subject of administration. For example, 50 to 200 µl per eye is administered by injection into the anterior chamber of human weighing 25 to 300 Kg, age 16 to 100 years, with bullous keratopathy as a symptom. The substrate for cell transplantation of the present invention suspends cells or cell spheroids and can be administered into the anterior chamber as a cell suspension. It is safe and does not cause side effects such as an increase in intraocular pressure or significant inflammation. The disease suitable for transplantation is a disease requiring corneal endothelium transplantation. As such disease, bullous keratopathy, circular conecornea, corneitis, chemical burns of the cornea, corneal stromal dystrophy, corneal edema, corneal leukoma, and the like can be mentioned, though not limited thereto.

### 2. Production methods of cell seeding agent or substrate for cell transplantation

The cell seeding agent or substrate for cell transplantation of the present invention contains a medium to dissolve and/or dilute a phenolphthalein derivative and maintain cell survival.

A medium for the cell seeding agent is not particularly limited as long as it functions to proliferate and engraft a cell after seeding, and physiological saline, various physiological buffers (e.g., PBS, HBSS, etc.), and those based on various basal media for cell culture may be used. The basal medium is not particularly limited as long as it can be used for culturing animal cells, and includes, for example, MEM medium, BME medium, BGJb medium, CMRL 1066 medium, Glasgow MEM medium, Improved MEM Zinc Option medium, IMDM medium, medium 199 medium, Eagle MEM medium, aMEM medium, DMEM medium, ham medium (e.g., F10, F12), RPMI 1640 medium, Fischer's medium, and a mixed medium thereof. These media are commercially available. Furthermore, these media can be a serum-containing medium or serum-free medium, preferably a serum-free medium. When the medium to be used in the present invention is a serum-containing medium, mammalian sera such as bovine serum, fetal bovine serum and the like can be used. The concentration of the serum in a medium is 0.1 to 20% (v/v), preferably 1 to 10% (v/v).

While the medium for the substrate for transplantation is not particularly limited as long as it is a solution suitable for intraocular administration, particularly administration into the anterior chamber, a nonirritating isotonized culture medium, saline, buffer and the like are used. Preferably, a culture medium similar to one used for culturing the cultured corneal endothelial cell or corneal endothelial substitute cell is used. The substrate for transplantation can contain various components as necessary besides phenolphthalein derivative. While the component is not particularly limited as long as it is useful for differentiation of the cultured corneal endothelial cell or corneal endothelial substitute cell, that has been administered (transplanted) into the anterior chamber, into corneal endothelial cell layer without falling off, it is preferable to add insulin or insulin-like growth factor. Also, retinoic acid, EGF and bFGF are also preferable components to be added.

The cell seeding agent and substrate for cell transplantation of the present invention can be prepared by adding and dissolving a phenolphthalein derivative in the above-mentioned medium. The addition concentration of the phenolphthalein derivative to the medium is the same as the concentration of a phenolphthalein derivative contained in the cell seeding agent or substrate for cell transplantation of the present invention as described in the above-mentioned "1. Cell seeding agent and substrate for cell transplantation", and is generally 1 to 20 mg/L, preferably 1 to 15 mg/L, more preferably 5 to 10 mg/L.

### [Example]

The present invention is described in detail in the following by referring to Examples, which are not to be construed as limitative. Unless particularly specified, the reagents and materials to be used are commercially available. Abbreviations used in the present specification are the same as those generally used in the art, unless otherwise specified.

### (Material and method)

### Reference Example 1: Influence of ROCK inhibitor on tight junction formation

An influence of a ROCK inhibitor during the passage operation in the differentiation induction step of iPS cell-derived corneal endothelial substitute cells was investigated. According to Example 1 and Experimental Example 2 of Patent Literature 3 (10 µM Y-27632 was used as ROCK inhibitor instead of fasudil), the expression of ZO-1 was investigated. The results are shown in Fig. 1. It was found that the use of a ROCK inhibitor inhibits the formation of tight junction and causes transformation that appears to be epithelial-to-mesenchymal transition. This result revealed that it is necessary to develop a cell seeding agent that does not contain a ROCK inhibitor.

### Example 1: Influence of phenolphthalein derivatives on engraftment and survival of cell after seeding

### (Material and method)

### 1. Medium for inducing corneal endothelial substitute cell

As a basal medium, DMEM/F12 medium (NACALAI TESQUE, INC., #08460-95) was used. As additives, N-2 MAX Media Supplement (100X) (R&D System Inc., #AR009), ascorbic acid (Fuso Pharmaceutical Industries, Ltd.; ascorbic acid injection (50 µg/mL)), IGF1 (OrphanPacific, Inc.; Somazon injection (20 ng/mL)), KGF (FUJIFILM Wako Pure Chemical Corporation, #119-00661 (5 ng/mL)), LIF (Sigma Aldrich, #L5283 (1 ng/mL)), IL6 (FUJIFILM Wako Pure Chemical Corporation; #099-04631 (1 ng/ml)), adrenaline (DAIICHI SANKYO COMPANY, LIMITED, Bosmin injection (0.5 µg/mL)), dexamethasone (KYOWA CRITICARE, Orgadrone injection (38 ng/mL)), and aldosterone (Sigma Aldrich, ##A9477 (720 ng/mL) were added to the DMEM/F12 basal medium, and a culture dish or culture flask coated with laminin 511-E8 fragment (iMatrix 511, Nippi Co., Ltd.) at a concentration of 3 ug/ml as a scaffold material was used.

### 2. Induction protocol for corneal endothelial substitute cell

Performed according to the method described in Example of Patent Literature 3. Specifically, as described below.

Y-27632 (wako/039-24591) was added to iPS cell medium AK03N (Ajinomoto Co., Inc.) at a concentration of 10 µM (hereinafter referred to as AK03N+Y medium). A 6-well culture plate (Greiner 657160) was coated with iMatrix 511 at a concentration of 6 ug/ml. iPS cells (FF-I01s04 strain) were suspended in AK03N+Y medium at a density of 1.0×10⁴ cells/cm² and seeded in this 6-well plate. Culture was started in a CO₂ incubator at 37°C, and the medium was replaced the next day with AK03N medium not containing Y-27632. Thereafter, the culture medium was exchanged at a frequency of once every 2 to 3 days, and once a week, the cells were passaged to an iMatrix 511-coated 6-well plate using TrypLE-Select (Thermo Fisher Scientific) at the same density as above. For induction into corneal endothelial substitute cells, iPS cells of not less than 3 passages and not more than 29 passages were used.

When starting the induction, iPS cells were collected from the 6-well plate using TrypLE-Select and suspended in the above-mentioned medium for inducing corneal endothelial substitute cells. A 35 mm or 100 mm culture dish was coated with iMatrix 511 at a concentration of 3 µg/ml, and suspended iPS cells were seeded at a density of 2.0×10⁴ cells/cm². Culture was started in a CO₂ incubator at 37°C, and thereafter, the medium was replaced at a frequency of once every 2 to 3 days.

### Example 2: Influence of phenolphthalein derivative on passage operation in differentiation induction step of iPS cell-derived corneal endothelial substitute cell

When induction into corneal endothelial substitute cell was started, iPS cells recovered using TrypLE-Select (Thermo Fisher Scientific) were suspended in a phenol red-added cell seeding agent or phenol red no-addition cell seeding agent. A 100 mm culture dish (Sumitomo Bakelite Co., Ltd.) was coated with iMatrix 511 at a concentration of 3 µg/ml, and iPS cells suspended in each cell seeding agent were seeded at a density of 1.0×10⁴ cells/cm². Culture was started in a CO₂ incubator at 37°C, and thereafter, the medium was replaced with phenol red-added corneal endothelial substitute cell induction medium or phenol red no-addition corneal endothelial substitute cell induction medium at a frequency of once every 2 to 3 days thereafter.

Culture was performed for 14 days under the above-mentioned conditions in each medium for inducing corneal endothelial substitute cells. After 3, 5, and 7 days of culture, the state of the cells was confirmed under a microscope (Fig. 2A). After culturing for 14 days, for the groups in which cell engraftment was observed under a microscope, the cells were collected and the number of cells was counted. The cells were collected after removing the culture supernatant, washed twice using DPBS(-) (Thermo Fisher Scientific), detached using Accutase (Innovative Cell Technologies), and collected into a centrifuge tube. The cells collected into the centrifuge tube were separated by centrifugation, and after adjusting the cell concentration using a medium and the like, the cell concentration and survival rate were measured using an automatic cell counter (ChemoMetec) (Fig. 2B).

### (Results)

The number of cells engrafted after cell seeding during the passage operation in the differentiation induction step of iPS cell-derived corneal endothelial substitute cells was investigated, and which components in the cell seeding agent had a critical influence was searched. As a result, phenol red was found to be a component with a critical influence.

Fig. 2(A) shows the results of observing cells on the 3rd, 5th, and 7th day of culturing in a medium added with or without phenol red after seeding. Engraftment was clearly better when cultured in a medium supplemented with phenol red after seeding.

Furthermore, by comparison of the number of recovered viable cells and survival rate on day 14 after culturing in a medium added with or without phenol red, it was clear that culturing in a medium supplemented with phenol red was clearly superior in the number of recovered cells and survival rate.

### Example 3: Influence of phenolphthalein derivative on engraftment of post-transplantation cells and corneal edema

### (Material and method)

A cell suspension of iPS cell-derived corneal endothelial substitute cells (8×10⁵ cells/160 ul) obtained in Example 1 was transplanted to the anterior chamber of a Macaca fascicularis bullous keratopathy model (conducted by SHIN NIPPON BIOMEDICAL LABORATORIES, LTD.). Two types of substrates for transplantation (containing insulin, insulin-like growth factor, and the like) were prepared: one with and one without the addition of phenol red.

The corneal endothelial substitute cell suspension was transplanted into the anterior chamber of the eye by the following procedure.
(1) Corneal endothelial substitute cells suspended in two types of substrates for transplantation, with and without phenol red added, were injected into the anterior chamber using a 27-gauge syringe.
(2) Macaca fascicularis was placed face down for 3 hr.
(3) After 28 days of observation, the eyeballs were collected and observed macroscopically and microscopically.

Formation of a corneal endothelial substitute cell layer and corneal thickness were measured for the transplanted eye.

Corneal thickness was measured using a corneal thickness measuring device (Tomey Corporation; SP-100).

### (Results)

The results are shown in Fig. 3 and Fig. 4. When transplanted using a substrate for transplantation containing phenol red, it was confirmed that corneal endothelial substitute cells were engrafted on the posterior cornea and spread uniformly. On the other hand, when transplantation was performed using a substrate for transplantation to which phenol red was not added, the engraftment of transplanted cells was biased (Fig. 4). In addition, a decrease in corneal thickness was observed when a substrate for transplantation to which phenol red was added was used, and therefore, it was confirmed that corneal edema was improved. No significant decrease in corneal thickness was confirmed when a substrate for transplantation to which phenol red was not added was used.

### Example 4: Phenolphthaleins corneal endothelial substitute cell induction protocol

### 1. iPS cell expansion culture (thawing)

T12.5 flask (Corning Incorporated/353107) was coated with iMatrix511 (MATRIXOME, Inc./892005) at a concentration of 6 µg/mL. iPS cells (FF-I01s04 strain) were suspended in AK03N+Y medium at a density of 8.0×10³ cells/cm², and were seeded into the T12.5 flask. Culture was started in a CO₂ incubator at 37°C, and the medium was replaced the next day with AK03N medium not containing Y-27632. After a gap of 2 days, medium exchange was performed for 2 consecutive days, and passage was performed on the 6th day of culture.

A T25 flask (Sumitomo Bakelite Co., Ltd./MS-23050) was coated with iMatrix511 (MATRIXOME, Inc./892005) at a concentration of 6 µg/mL. iPS cells were suspended in AK03N+Y medium at a density of 2.2×10³ cells/cm², and were seeded into the T25 flask. Culture was started in a CO₂ incubator at 37°C, and the medium was replaced the next day with AK03N medium not containing Y-27632. After a gap of 2 days, medium exchange was performed for 2 consecutive days, and passage was performed on the 6th day of culture.

### 2. Preparation of phenol red-free corneal endothelial substitute cell induction medium

DMEM/F12 non phenol red (Thermo Fisher Scientific, Inc./11039-021) was used as the basal medium, and a phenol red-free corneal endothelial substitute cell induction medium was prepared (hereinafter also to be referred to as phenol red-free medium in this Example). To the basal medium (500 mL) were added, as additives, ITS-Supplement (Ventria/777ITS091), IGF1 (OrphanPacific, Inc.; Somazone for injection/858100075, 1 mg/mL), LIF (FUJIFILM Wako Pure Chemical Corporation/125-06661), IL-6 (Miltenyi Biotec/170-076-161), IL-11 (Pepro tech/AF-200-11), TNFα (Miltenyi Biotec/170-076-178), and water-soluble HYDROCORTONE Injection (Nichi-Iko Pharmaceutical Co., Ltd./27126333) described in the following Table 1.

**[Table 1]**

| reagent name | amount added | final concentration |
|---|---|---|
| LIF | 200 µL | 10 ng/mL |
| IL-11 | 250 µL | 5 ng/mL |
| IL-6 | 250 µL | 10 ng/mL |
| TNFα | 100 µL | 10 ng/mL |
| ITS-Supplement | 5 mL | 10 mL/L |
| IGF1 | 5 µL | 10 ng/mL |
| HYDROCORTONE | 20 µL | 200 nM |

### 3. Preparation of phenolphthaleins solution

The phenolphthalein derivatives shown in the following Table 2, B to D, were dissolved in advance in ethanol to a concentration of 6.6×10⁻⁶ mol/mL. 30 mL each of the phenol red-free corneal endothelial substitute cell induction medium prepared in the above-mentioned 2. was dispensed into four 50 mL tubes, and 40.5 µL of A phenolsulfonphthalein injection 0.6% (AFP) was added to one of the four tubes. Furthermore, 100 µL of B cresol red solution with adjusted concentration was added to one of the remaining three tubes. Similarly, C thymol blue solution and D bromocresol purple solution were respectively added to the remaining two tubes. A total of four different phenolphthalein derivative-containing media were prepared and used as phenolphthalein derivative-added cell seeding agents. Each seeding agent was adjusted to have a phenolphthalein derivative concentration of 0.22 mM.

**[Table 2]**

| | | |
|---|---|---|
| A | phenolsulfonphthalein intramuscular injection 0.6% (phenol red) | Alfresa Pharma Corporation |
| B | cresol red | TGI/C0406 |
| C | thymol blue | NACALAI TESQUE, INC./33908-81 |
| D | bromocresol purple | NACALAI TESQUE, INC./05612-11 |

### 4. Differentiation induction (seeding density fixed at 1.04×10⁴ cells/cm²)

90 µL of laminin 511-E8 fragment (iMatrix511MG) was added to 30 mL of DPBS (-), added to a 6-well plate at 1.5 mL/well for coating same at a concentration of 3 ug/ml as a scaffold material, and the plate was left standing at 37°C for 1 hr or longer. The supernatant was removed and various phenolphthalein derivative-added cell seeding agents A to D prepared in the above-mentioned 3. were added at 2 mL/well, and the mixture was incubated at 37°C. After the iPS cell expansion culture in the above-mentioned 1., the recovered iPS cells were centrifuged at 140×g, 25°C for 5 min, the supernatant was removed, and the cells were suspended in various phenolphthalein derivative-added cell seeding agents A to D, or phenolphthalein-free medium and seeded in iMatrix-coated 6-well plates at a seeding density of 1.04×10⁴ cells/cm². The cells were incubated in a CO₂ incubator at 37°C. The time of seeding was set as day 0, the first medium exchange was performed at 2 mL/well on the 3rd day, then the medium was changed every other day thereafter, and on the 7th day after seeding, the cells were fixed and stained.

### 5. Differentiation induction (changing seeding density)

After coating with iMatrix in the same manner as in the above-mentioned 4., the supernatant was removed and a normal differentiation induction medium (phenol red-containing DMEM/F12 (Thermo Fisher/11330057) with the additives listed in Table 1 above) was used as a positive control (posicon), phenol red-free medium using DMEM/F12 non phenol red (Thermo Fisher/11039-021) instead of DMEM/F12 as a negative control (negacon), A Phenol red-added cell seeding agent, and B Cresol red-added cell seeding agent were each added to 5 wells at 2 mL/well and incubated at 37°C. After expansion culture in the same manner as the above-mentioned 1., the recovered iPS cells were centrifuged at 140×g, 25°C for 5 min, the supernatant was removed, and the cells were suspended in posicon, negacon, A phenol red-added cell seeding agent-added medium, B cresol red-added cell seeding agent-added medium, and seeded in each of the wells of A phenol red-added cell seeding agent, B cresol red-added cell seeding agent, posicon, and negacon in a 6-well plate coated with iMatrix at a seeding density 1.04×10³ cells/cm², 5.02×10³ cells/cm², 1.04×10⁴ cells/cm², 5.02×10⁵ cells/cm², and 1.04×10⁵ cells/cm². The cells were incubated in a CO₂ incubator at 37°C. The time of seeding was set as day 0, the first medium exchange was performed at 2 mL/well on the 3rd day, then the medium was changed every other day thereafter, and on the 7th day after seeding, the cells were fixed and stained.

### 6. Results

In a phenol red-free medium, no engraftment occurred when seeded not more than the specified seeding density of 1.04×10⁴ cells/cm².

In the medium supplemented with phenol red and cresol red, cell engraftment was confirmed at a seeding number lower than the specified cell seeding density.

Engraftment was also confirmed in thymol blue-containing medium and bromocresol purple-containing medium when seeded at 1.04×10⁴ cells/cm².

### [Industrial Applicability]

According to the present invention, it is possible to perform passage operation without causing cell transformation and efficiently engraft cells. Large-scale production of cultured corneal endothelial cells and corneal endothelial substitute cells can be achieved, and the engraftment rate of transplanted cells is increased, thereby making it possible to improve treatment effects.

This application is based on a US provisional patent application No. 63/219,101 filed in the US (filing date: July 7, 2021), the contents of which are incorporated in full herein.

## Claims

1. A seeding agent for promoting the engraftment of a cultured corneal endothelial cell or a corneal endothelial substitute cell, comprising a phenolphthalein derivative.

2. A seeding agent for promoting the engraftment of a corneal endothelial substitute cell, comprising a phenolphthalein derivative.

3. The agent according to claim 2, wherein the corneal endothelial substitute cell is derived from a stem cell.

4. The agent according to claim 3, wherein the stem cell is a pluripotent stem cell.

5. The agent according to claim 4, wherein the pluripotent stem cell is derived from an iPS cell (including a cell imparted with an additional function by gene editing or gene transfer).

6. The agent according to any one of claims 1 to 5, wherein the phenolphthalein derivative is selected from the group consisting of phenolsulfonphthalein (phenol red), isovalerylphenolphthalein, acetylphenolphthalein, phenolphthalein dibutyrate, phenolphthalein diphosphate, phenolphthalein disulfate, phenolphthalein glucuronic acid, phenolphthalein mono-β-glucosiduronic acid, phenolphthalein mono-β-glucuronic acid, phenolphthalein mono-phosphate, cresol red, thymol blue, and bromocresol purple.

7. The agent according to any one of claims 1 to 6, wherein the phenolphthalein derivative is phenol red.

8. The agent according to claim 7, wherein a concentration of the phenol red is 1 to 20 mg/L.

9. The agent according to any one of claims 1 to 6, wherein the phenolphthalein derivative is cresol red.

10. The agent according to claim 9, wherein a concentration of the cresol red is 1 to 20 mg/L.

11. The agent according to any one of claims 1 to 10, which is free of a ROCK inhibitor.

12. A cell suspension comprising the agent according to any one of claims 2 to 11 and a corneal endothelial substitute cell.

13. The cell suspension according to claim 12, wherein the seeding is performed at a seeding density of not less than 1.04×10³ cells/cm² and less than 1.04×10⁴ cells/cm².

14. A substrate for the transplantation of a cultured corneal endothelial cell or a corneal endothelial substitute cell, comprising a phenolphthalein derivative.

15. A substrate for the transplantation of a corneal endothelial substitute cell, comprising a phenolphthalein derivative.

16. The substrate according to claim 14 or 15, wherein the corneal endothelial substitute cell is derived from a stem cell.

17. The substrate according to claim 16, wherein the stem cell is a pluripotent stem cell.

18. The substrate according to claim 17, wherein the pluripotent stem cell is derived from an iPS cell (including a cell imparted with an additional function by gene editing or gene transfer).

19. The substrate according to any one of claims 14 to 18, wherein the phenolphthalein derivative is selected from the group consisting of phenolsulfonphthalein (phenol red), phenolphthalein, bromophenol blue, isovalerylphenolphthalein, acetylphenolphthalein, phenolphthalein dibutyrate, phenolphthalein diphosphate, phenolphthalein disulfate, phenolphthalein glucuronic acid, phenolphthalein mono-β-glucosiduronic acid, phenolphthalein mono-β-glucuronic acid, phenolphthalein mono-phosphate, cresol red, thymol blue, and bromocresol purple.

20. The substrate according to any one of claims 14 to 19, wherein the phenolphthalein derivative is phenol red.

21. The substrate according to claim 20, wherein a concentration of the phenol red is 1 to 20 mg/L.

22. The substrate according to any one of claims 14 to 19, wherein the phenolphthalein derivative is cresol red.

23. The substrate according to claim 22, wherein a concentration of the cresol red is 1 to 20 mg/L.

24. The substrate according to any one of claims 14 to 23, which is free of a ROCK inhibitor.

25. A cell suspension comprising the substrate according to any one of claims 14 to 24 and a corneal endothelial substitute cell.

26. The cell suspension according to claim 25, which is for transplantation into the anterior chamber.

27. The cell suspension according to claim 26, wherein a concentration of the cell is 0.5 to 10×10⁶ cells/mL.
